(19) Europäisches Patentamt
European Patent Office
Office européen des brevets

(11) Veröffentlichungsnummer **0 005 830**
**B1**

(12) # EUROPÄISCHE PATENTSCHRIFT

(45) Veröffentlichungstag der Patentschrift: **22.09.82**

(21) Anmeldenummer: **79101657.9**

(22) Anmeldetag: **30.05.79**

(51) Int. Cl.³: **C 07 D 501/36,**
A 61 K 31/545

(54) **Cephalosporinderivate, deren Herstellung und die entsprechenden pharmazeutischen Präparate.**

(30) Priorität: **30.05.78 CH 5882/78**
**08.03.79 CH 2248/79**

(43) Veröffentlichungstag der Anmeldung:
**12.12.79 Patentblatt 79/25**

(45) Bekanntmachung des Hinweises auf die Patenterteilung:
**22.09.82 Patentblatt 82/38**

(84) Benannte Vertragsstaaten:
**AT BE CH DE FR IT LU NL SE**

(56) Entgegenhaltungen:
DE - A - 2 534 071
DE - A - 2 707 565
DE - A - 2 713 272
DE - A - 2 715 385
DE - A - 2 738 712

Die Akte enthält technische Angaben, die nach dem Eingang der Anmeldung eingereicht wurden und die nicht in dieser Patentschrift enthalten sind.

(73) Patentinhaber: **F. HOFFMANN-LA ROCHE & CO. Aktiengesellschaft**
**CH-4002 Basel (CH)**

(72) Erfinder: **Montavon, Marc, Dr.**
**Realpstrasse 72**
**CH-4054 Basel (CH)**
Erfinder: **Reiner, Roland, Dr.**
**Rheinfelderstrasse 8**
**CH-4058 Basel (CH)**

(74) Vertreter: **Lederer, Franz, Dr. et al,**
**Patentanwälte Dr. Franz Lederer Reiner F. Meyer**
**Lucile-Grahn-Strasse 22**
**D-8000 München 80 (DE)**

EP 0 005 830 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# Cephalosporinderivate, deren Herstellung und die entsprechenden pharmazeutischen Präparate

Die vorliegende Erfindung betrifft neue Cephalosporinderivate, und zwar ein Cephalosporinderivat der allgemeinen Formel

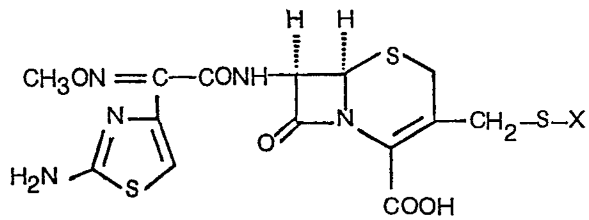

I

in der X die 1,2,5,6-Tetrahydro-2-methyl-5,6-dioxo-as-triazin-3-ylgruppe oder deren entsprechende tautomere Form, die 2,5-Dihydro-6-hydroxy-2-methyl-5-oxo-as-triazin-3-ylgruppe, bedeutet, sowie leicht hydrolysierbare Ester/Aether und Salze dieser Verbindung und Hydrate der Verbindung der Formel I bzw. von deren Estern/Aethern und Salzen.

Als leicht hydrolysierbare Ester der Verbindung Formel I sind Verbindungen entsprechend der Formel I zu verstehen, deren Carboxygruppe in Form einer leicht hydrolysierbaren Estergruppe vorliegt. Beispiele solcher Ester, die herkömmlicher, Art sein können, sind die niederen Alkanoyloxyalkylester, z.B. der Acetoxymethyl-, Pivaloyloxymethyl-, 1-Acetoxyäthyl- und 1-Pivaloyloxyäthylester; die niederen Alkoxycarbonyloxyalkylester, z.B. der Methoxycarbonyloxymethyl-, 1-Aethoxycarbonyloxyäthyl- und 1-Isopropoxycarbonyloxyäthylester; die Lactonylester, z.B. der Phthalidyl- und Thiophthalidylester; die niederen Alkoxymethylester, z.B. der Methoxymethylester; und die niederen Alkanoylaminomethylester, z.B. der Acetamidomethylester. Auch andere Ester, z.B. die Benzyl- und Cyanmethylester, können brauchbar sein.

Als leicht hydrolysierbare Aether der Verbindung der Formel I sind Verbindungen entsprechend der Formel I zu verstehen, worin X die 2,5-Dihydro-6-hydroxy-2-methyl-5-oxo-as-triazin-3-ylgruppe bedeutet, deren enolische OH-Gruppe in Form einer leicht hydrolysierbaren Aethergruppe vorliegt. Als Aethergruppen kommen die gleichen Gruppen in Betracht, wie sie oben bereits für die leicht hydrolysierbaren Estergruppen erwähnt wurden. Vertreter solcher Aether sind also beispielsweise die niederen Alkanoyloxyalkyläther, z.B. der Acetoxymethyl-, Pivaloyloxymethyl-, 1-Acetoxyäthyl- und 1-Pivaloyloxyäthyläther; die niederen Alkoxycarbonyloxyalkyläther, z.B. der Methoxycarbonyloxymethyl-, 1-Aethoxycarbonyloxyäthyl- und 1-Isopropoxycarbonyloxyäthyläther; die Lactonyläther, z.B. der Phthalidyl- und Thiophthalidyläther; die niederen Alkoxymethyläther, z.B. der Methoxymethyläther; und die niederen Alkanoylaminomethyläther, z.B. der Acetamidomethyläther.

Beispiele von Salzen der Verbindung der Formel I sind Alkalimetallsalze, wie das Natrium- und Kaliumsalz; das Ammoniumsalz; Erdalkalimetallsalze, wie das Calciumsalz; Salze mit organischen Basen, wie Salze mit Aminen, z.B. Salze mit N-Aethyl-piperidin, Procain, Dibenzylamin, N,N'-Dibenzyläthyläthylendiamin, Alkylaminen oder Dialkylaminen, sowie Salze mit Aminosäuren, wie z.B. Salze mit Arginin oder Lysin. Die Salze können Monosalze oder auch Disalze sein. Die zweite Salzbildung kann in Verbindungen mit dem Hydroxyrest der 2,5-Dihydro-6-hydroxy-2-methyl-5-oxo-as-triazin-3-ylgruppe auftreten.

Die Verbindung der Formel I bildet ebenfalls Additionssalze mit organischen oder anorganischen Säuren. Beispiele solcher Salze sind Hydrohalogenide, beispielsweise Hydrochloride, Hydrobromide, Hydrojodide, sowie andere Mineralsäuresalze, wie Sulfate, Nitrate, Phosphate und dgl., Alkyl- und Mono-arylsulfonate, wie Aethansulfonate, Toluolsulfonate, Benzolsulfonate und dgl. und auch andere organische Säuresalze, wie Acetate, Tartrate, Maleate, Citrate, Benzoate, Salicylate, Ascorbate und dgl.

Die Verbindung der Formel I sowie deren Salze und leicht hydrolysierbare Ester/Aether können hydratisiert sein. Die Hydratisierung kann im Zuge des Herstellungsverfahrens erfolgen oder allmählich als Folge hygrokopischer Eigenschaften eines zunächst wasserfreien Produktes auftreten.

Die erfindungsgemässen Produkte können in der synisomeren Form

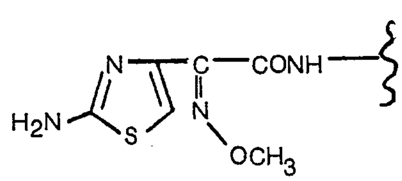

oder in der anti-isomeren Form

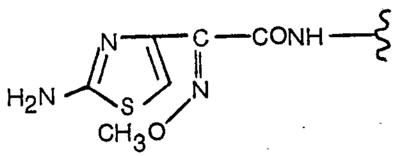

bzw. als Gemische dieser beiden Formen vorliegen. Bevorzugt ist die syn-isomere Form bzw. Gemische, in denen die syn-isomere Form überwiegt.

Bevorzugte Produkte sind

(6R, 7R) - 7 - [2 - (2 - Amino - 4 - thiazolyl) - 2 - (Z - methoxyimino)acetamido] - 3 - /[(2,5 - dihydro - 6 - hydroxy - 2 - methyl - 5 - oxo - as - triazin - 3 - yl)thio] - methyl/ - 8 - oxo - 5 - thia - 1 - azabicyclo[4.2.0]oct - 2 - en - 2 - carbonsäure und deren Salze sowie die entsprechenden Hydrate.

Die obigen Cephalosporinderivate werden erfindungsgemäss dadurch hergestellt, dass man

a) in einer Verbindung der allgemeinen Formel

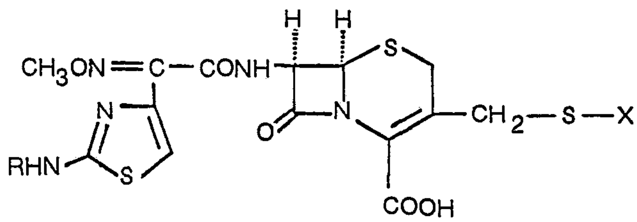

II

in der X die oben gegebene Bedeutung hat, R eine abspaltbare Schutzgruppe darstellt und die Carboxygruppe in geschützter Form vorliegen kann, die Schutzgruppe R, gegebenenfalls auch eine allenfalls vorhandene Carboxyschutzgruppe, abspaltet oder dass man

b) zur Herstellung eines leicht hydrolysierbaren Esters bzw. Aethers der Verbindung der Formel I diese einer entsprechenden Veresterung bzw. Verätherung unterwirft oder dass man

c) zur Herstellung von Salzen oder Hydraten der Verbindung der Formel I bzw. Hydraten dieser Salze die Verbindung der Formel I in ein Salz oder Hydrat bzw. ein Hydrat dieses Salzes überführt.

Die in der Ausgangsverbindung der Formel II vorhandene Carboxygruppe kann wahlweise geschützt sein, z.B. durch Veresterung zu einem leicht spaltbaren Ester, wie dem Silylester, z.B. dem Trimethylsilylester. Es kommen auch die oben erläuterten, leicht hydrolysierbaren Ester in Betracht. Die Carboxygruppe kann auch durch Salzbildung mit einer anorganischen oder tertiären organischen Base, wie Triäthylamin, geschützt werden. Mögliche R-Schutzgruppen sind beispielsweise sauer-hydrolytisch abspaltbare Schutzgruppen, wie z.B. t-Butoxycarbonyl oder Trityl, oder auch basisch-hydrolytisch abspaltbare Schutzgruppen, wie z.B. Trifluoracetyl. Bevorzugte R-Schutzgruppen sind Chlor-, Brom- und Jodacetyl, insbesondere Chloracetyl. Letztere Schutzgruppen können durch Behandeln mit Thioharnstoff abgespalten werden.

Die Ausgangsverbindungen der Formel II Sind Gegenstand der Anmeldung EP - A - 8106777, 6 und können z.B. durch N-Acylierung der entsprechenden 7-Aminoverbindung hergestellt werden, und zwar indem man eine Verbindung der allgemeinen Formel

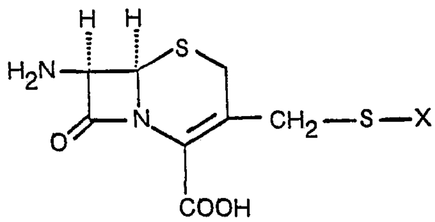

III

in der X die oben gegebene Bedeutung hat und die Carboxygruppe und/oder die Aminogruppe in geschützter Form vorliegen kann, mit einer Säure der allgemeinen Formel

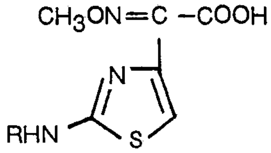

IV

in der R die oben gegebene Bedeutung hat, oder mit einem reaktionsfähigen funktionellen Derivat

dieser Säure umsetzt und eine allenfalls vorhandene Carboxyschutzgruppe gegebenenfalls abspaltet.

Die in der 7-Aminoverbindung der Formel III vorhandene Carboxygruppe kann wahlweise geschützt sein, und zwar in der oben für die herzustellende Ausgangsverbindung der Formel II erläuterten Weise. Die Aminogruppe der Verbindung der Formel III kann z.B. durch eine Silylschutzgruppe, wie Trimethylsilyl, geschützt sein.

Als reaktionsfähige funktionelle Derivate von Säuren der Formel IV kommen z.B. Halogenide, d.h. Chloride, Bromide und Fluoride; Azide; Anhydride, insbesondere gemischte Anhydride mit stärkeren Säuren; reaktionsfähige Ester, z.B. N-Hydroxysuccinimidester, und Amide, z.B. Imidazolide, in Betracht.

Die Umsetzung der 7-Aminoverbindung der Formel III mit der Säure der Formel IV oder einem reaktionsfähigen funktionellen Derivat davon kann in an sich bekannter Weise durchgeführt werden. So kann man z.B. eine freie Säure der Formel IV mit einem der erwähnten Ester entsprechend Formel III mittels eines Carbodiimids, wie Dicyclohexylcarbodiimid, in einem inerten Lösungsmittel, wie Essigester, Acetonitril, Dioxan, Chloroform, Methylenchlorid, Benzol oder Dimethylformamid, kondensieren und anschliessend die Estergruppe abspalten. Anstelle von Carbodiimiden lassen sich als Kondensationsmittel auch Oxazoliumsalze, z.B. N-Aethyl-5-phenyl-isoxazolium-3'-sulfonat, verwenden.

Nach einer anderen Ausführungsform setzt man ein Salz einer Säure der Formel III, z.B. ein Trialkylammoniumsalz, wie das Triäthylammoniumsalz, mit einem reaktionsfähigen funktionellen Derivat einer Säure der Formel IV wie oben erwähnt in einem inerten Lösungsmittel, z.B. einem der oben genannten, um.

Nach einer weiteren Ausführungsform wird ein Säurehalogenid, vorzugsweise das Chlorid einer Säure der Formel IV mit dem Amin der Formel III umgesetzt. Die Umsetzung erfolgt vorzugsweise in Gegenwart eines säurebindenden Mittels, z.B. in Gegenwart von wässrigem Alkali, vorzugsweise Natronlauge, oder auch in Gegenwart eines Alkalimetallcarbonats, wie Kaliumcarbonat, oder in Gegenwart eines nieder-alkylierten Amins, wie Triäthylamin. Als Lösungsmittel wird vorzugsweise Wasser verwendet, ggfs. im Gemisch mit einem inerten organischen Lösungsmittel, wie Tetrahydrofuran oder Dioxan. Es kann auch in einem aprotischen organischen Lösungsmittel, wie z.B. Dimethylformamid, Dimethylsulfoxid oder Hexamethylphosphorsäuretriamid, gearbeitet werden. Bei Verwendung von silylierten Ausgangsverbindungen der Formel III wird in wasserfreiem Medium gearbeitet.

Die Umsetzung der 7-Aminoverbindung der Formel III mit der Säure der Formel IV oder einem reaktionsfähigen funktionellen Derivat davon kann zweckmässig bei Temperaturen zwischen etwa —40°C und Zimmertemperatur, beispielsweise bei etwa 0—10°C, erfolgen.

Gemäss Verfahrensvariante a) des erfindungsgemässen Verfahrens wird die Aminoschutzgruppe R einer Ausgangsverbindung der Formel II abgespalten. Durch saure Hydrolyse abspaltbare Schutzgruppen werden vorzugsweise mit Hilfe einer niederen Alkancarbonsäure, die ggfs. halogeniert sein kann, entfernt. Insbesondere verwendet man Ameisensäure oder Trifluoressigsäure. Die Temperatur ist in der Regel Raumtemperatur, obwohl auch leicht erhöhte bzw. leicht erniedrigte Temperatur angewendet werden kann, z.B. im Bereiche von etwa 0°C bis +40°C. Alkalisch abspaltbare Schutzgruppen werden im allgemeinen mit verdünnter wässriger Lauge bei 0°C bis 30°C hydrolysiert. Die Chloracetyl-, Bromacetyl- und Jodacetyl-Schutzgruppen können mittels Thioharnstoff in saurem, neutralem oder alkalischem Milieu bei etwa 0—30°C abgespalten werden. Hydrogenolytische Abspaltung (z.B. Abspaltung von Benzyl) ist hier ungeeignet, da bei der Hydrogenolyse die Oximfunktion zur Aminogruppe reduziert wird.

Nach Durchführung der Verfahrensvariante a), kann, falls erwünscht, eine allenfalls im Reaktionsprodukt vorhandene Carboxyschutzgruppe abgespalten werden. Wenn die Schutzgruppe eine Silylgruppe darstellt (Silylester), kann diese Gruppe besonders leicht durch Behandeln des Umsetzungsproduktes mit Wasser abgespalten werden. Niedere Alkanoyloxyalkyl-, Alkoxycarbonyloxyalkyl-, Lactonyl-, Alkoxymethyl- und Alkanoylaminomethylester werden vorzugsweise enzymatisch mit Hilfe einer geeigneten Esterase (bei etwa 20—40°C) abgespalten. Wenn die Carboxylgruppe durch Salzbildung (z.B. mit Triäthylamin) geschützt ist, so kann die Abspaltung dieser salzbildenen Schutzgruppe durch Behandlung mit Säure erfolgen. Als Säure kann hierbei z.B. Salzsäure, Schwefelsäure, Phosphorsäure oder Citronensäure verwendet werden.

Die Carboxyschutzgruppe kann in der gleichen Weise wie soeben beschrieben auch vor der Abspaltung der Schutzgruppe R abgespalten werden.

Zur Herstellung der leicht hydrolysierbaren Ester der Carbonsäure der Formel I gemäss Variante b) wird die Carbonsäure vorzugsweise mit dem entsprechenden, die Estergruppe enthaltenden Halogenid, bevorzugt mit dem Jodid, umgesetzt. Die Reaktion kann mit Hilfe einer Base, z.B. einem Alkalimetallhydroxid oder -carbonat oder einem organischen Amin, wie Triäthylamin beschleunigt werden. Die 2,5-Dihydro-6-hydroxy-2-methyl-5-oxo-as-triazin-3-ylgruppe X mit ihrer enolischen Funktion wird unter Bildung eines entsprechenden, leicht hydrolysierbaren Aethers veräthert. (Die so erhaltenen, gleichzeitig veresterten bzw. verätherten Produkte werden vor- bzw. nachstehend "leicht hydrolysierbare Ester/Aether" genannt). Vorzugsweise verwendet man dabei einen Ueberschuss an dem entsprechenden Halogenid. Die Veresterungs/Verätherungsreaktion wird vorzugsweise in einem inerten organischen Lösungsmittel durchgeführt, wie Dimethylacetamid, Hexamethylphosphorsäuretriamid, Dimethylsulfoxid oder, vorzugsweise, Dimethylformamid. Die Temperatur liegt vorzugsweise im Bereiche von etwa 0—40°C.

Die Herstellung der Salze und Hydrate der Verbindung der Formel I bzw. der Hydrate dieser Salze kann in an sich bekannter Weise erfolgen, z.B. durch Umsetzung der Carbonsäure der Formel I mit einer äquivalenten Menge der gewünschten Base, zweckmässig in einem Lösungsmittel, wie Wasser oder in einem organischen Lösungsmittel, wie Aethanol Methanol, Aceton und anderen mehr. Bei Verwendung eines zweiten Aequivalents an Base erfolgt Salzbildung auch an der tautomeren Enolform (2,5-Dihydro-6-hydroxy-2-methyl-5-oxo-as-triazin-3-ylgruppe X), wobei ein Disalz entsteht. Die Temperatur der Salzbildung ist nicht kritisch. Sie liegt im allgemeinen bei Raumtemperatur, kann aber auch leicht darüber oder darunter, etwa im Bereiche von 0°C bis +50°C, sein.

Die Herstellung der Hydrate erfolgt zumeist automatisch im Zuge des Herstellungsverfahrens oder als Folge hygroskopischer Eigenschaften eines zunächst wasserfreien Produktes. Zur gezielten Herstellung eines Hydrats kann ein ganz oder teilweise wasserfreies Produkt (Carbonsäure der Formel I bzw. Ester/Aether oder Salz davon) einer feuchten Atmosphäre, z.B. bei etwa +10°C bis +40°C, ausgesetzt werden.

Die oben verwendeten 7-Aminoverbindungen der Formel III können ausgehend von einer Verbindung der Formel

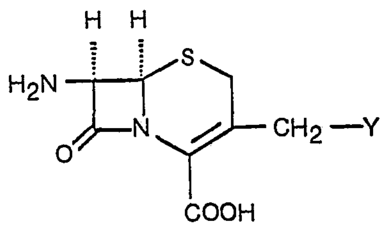

V

in der Y eine austretende Gruppe darstellt mit einem Thiol der allgemeinen Formel

$$HS—X \qquad VI$$

in der X die oben gegebene Bedeutung hat.

Als austretende Gruppe Y einer Verbindung der Formel V kommen beispielsweise Halogene, z.B. Chlor, Brom oder Jod, Acyloxyreste, z.B. niedere Alkanoyloxyreste, wie Acetoxy, niedere Alkyl- oder Arylsulfonyloxyreste, wie Mesyloxy oder Tosyloxy, oder der Azidorest in Frage.

Die Umsetzung der Verbindung der Formel V mit dem Thiol der Formel VI kann in an sich bekannter Weise, z.B. bei einer Temperatur zwischen etwa 40 und 80°C, zweckmässig bei etwa 60°C, in Wasser oder in einer Pufferlösung mit einem pH von etwa 6 bis 7, vorzugsweise 6,5, durchgeführt werden.

Ein allenfalls erhaltenes syn/anti-Gemisch der Verbindung der Formel I kann in die entsprechenden syn- und anti-Formen in üblicher Weise aufgetrennt werden, beispielsweise durch Umkristallisation oder durch chromatographische Methoden unter Verwendung eines geeigneten Lösungsmittels bzw. Lösungsmittelgemisches.

Die Verbindungen der Formel I und II sowie die entsprechenden leicht hydrolysierbaren Ester/Aether und Salze bzw. die Hydrate dieser Produkte sind antibiotisch, insbesondere bakterizid wirksam. Sie besitzen ein breites Wirkungsspektrum gegen Gram-positive und Gram-negative Mikroorganismen, einschliesslich β-Lactamase bildende Staphylokokken und verschiedende β-Lactamase-bildende Gram-negative Bakterien, wie. z.B. Pseudomonas aeruginosa, Haemophilus influenzae, Escherichia coli, Serratia marcescens, Proteus- und Klebsiella-Spezies.

Die Verbindungen der Formel I und II sowie die entsprechenden leicht hydrolysierbaren Ester/Aether und Salze bzw. die Hydrate dieser Produkte können zur Behandlung und Prophylaxe von Infektionskrankheiten verwendet werden. Für den Erwachsenen kommt eine Tagesdosis von etwa 0,1 g bis etwa 2 g in Betracht. Die parenterale Verabreichung der erfindungsgemässen Verbindungen ist besonders bevorzugt.

Zum Nachweis der antimikrobiellen Wirksamkeit der erwähnten Produkte wurden folgende repräsentative Vertreter getestet:

PRODUKT A:

(6R, 7R) - 7 - [2 - (2 - Amino - 4 - thiazolyl) - 2 - (Z - methoxyimino)acetamido] - 3 - /[(2,5 - dihydro - 6 - hydroxy - 2 - methyl - 5 - oxo - as - triazin - 3 - yl)thio]methyl/ - 8 - oxo - 5 - thia - 1 - azabicyclo[4.2.0] - oct - 2 - en - 2 - carbonsäure

PRODUKT B:

(6R, 7R) - 7 - /2 - [2 - (2 - Chloracetamido) - 4 - thiazolyl] - 2 - (Z - methoxyimino)acetamido/ - 3 -/ [(2,5 - dihydro - 6 - hydroxy - 2 - methyl - 5 - oxo - as - triazin - 3 - yl)thio]methyl/ - 8 - oxo - 5 - thia - 1 - azabicyclo[4.2.0]oct - 2 - en - 2 - carbonsäure

*Aktivität in vitro:* Mindesthemmkonzentration ($\mu g/ml$)

| | | A | B |
|---|---|---|---|
| Haemophilus influenzae | Stamm 1 | 0,08 | 1,2 |
| | Stamm 2 | 0,005 | 0,3 |
| | Stamm 3 | 0,005 | 0,16 |
| | Stamm 4 | 0,005 | 0,16 |
| | Stamm 5 | 0,0025 | 0,08 |
| | Stamm 6 | 0,0025 | 0,16 |
| | Stamm 7 | 0,0025 | 0,16 |
| Klebsiella pneumoniae | | 1,2 | 10 |
| Escherichia coli | Stamm 1 | 0,02 | 0,16 |
| | Stamm 2 | 0,6 | 5 |
| Proteus mirabilis | Stamm 1 | ≤0,01 | 0,08 |
| | Stamm 2 | ≤0,01 | 0,16 |
| Proteus vulgaris | | ≤0,01 | 0,16 |
| Proteus rettgeri | | ≤0,01 | 0,16 |
| Staphylococcus aureus | Stamm ATCC 6538 | 2,5 | 2,5 |
| | Penicillin-resistenter Stamm | 2,5 | 5 |
| Pseudomonas aeruginosa | Stamm 1 | 0,3 | 1,2 |
| | Stamm 2 | 10 | >80 |
| | Stamm 3 | 2,5 | 40 |
| | Stamm 4 | 5 | 80 |
| | Stamm 5 | 5 | 80 |
| | Stamm 6 | 10 | 80 |
| | Stamm 7 | 5 | 80 |
| Serratia marcescens | | 0,08 | 2,5 |

Aktivität in vivo

Gruppen von 5 Mäusen werden mit einer wässrigen Suspension von Escherichia coli intraperitoneal infiziert. Dreimal, d.h. 1 Stunde, 2 1/2 Stunden und 4 Stunden nach der Infektion, wird die Prüfsubstanz in physiologischer Kochsalzlösung subcutan appliziert. Die Zahl der überlebenden Tiere wird am 4. Tag bestimmt. Es werden verschiedene Dosierungen appliziert, und durch Interpolation wird diejenige Dosis bestimmt, bei der 50% der Versuchstiere überleben ($CD_{50}$, mg/kg).

| Prüfsubstanz | A | B |
|---|---|---|
| $CD_{50}$, mg/kg | ≤0,005 | 0,16 |

Toxizität

| Prüfsubstanz | | A | B |
|---|---|---|---|
| $LD_{50}$, mg/kg | i.v. | 250—500 | 250—500 |
| | s.c. | >4000 | 2000—4000 |
| | p.o. | >5000 | >5000 |

In der DE-A-27 15 385 und der DE-A-27 07 565 sind Cephalosporinderivate offenbart, welche, ähnlich wie die erfindungsgemässen Cephalosporine, eine 7-[2-(2-Amino-4-thiazolyl)-2-(Z-methoxyimino)-acetamido]-gruppe tragen. Die erfindungsgemässen Cephalosporine unterscheiden sich jedoch charakteristisch durch die Konfiguration des Substituenten in 3-Stellung, wodurch neue Verbindungen mit überraschenden Wirkungsvorteilen zur Verfügung gestellt werden. So zeigte ein Vergleich mit einer strukturell am ehesten vergleichbaren Verbindung aus dem oben angeführten Stand der Technik (einzige Verbindung, welche in 3-Stellung, ähnlich wie die erfindungsgemässen Cephalosporine, eine 6-gliedrige N-heterocyclische Thiomethylgruppe besitzt, u.zw. die (6-Methyl-1-oxidopyridazin-3-yl)-thiomethylgruppe), dass das erfindungsgemässe Cephalosporin der Formel I eine massive (mehr als 50mal stärkere) Wirkungsüberlegenheit im prophylaktischen in vivo Test an der Maus gegen die pathogenen Mikroorganismen Serratia marcescens, Klebsiella pneumoniae, Proteus mirabilis und Proteus vulgaris aufweist.

Die erfindungsgemässen Produkte können als Heilmittel z.B. in Form pharmazeutischer Präparate Verwendung finden, welche sie oder ihre Salze in Mischung mit einem für die enterale oder parenterale Applikation geeigneten pharmazeutischen, organischen oder anorganischen inerten Trägermaterial, wie z.B. Wasser, Gelatine, Gummi arabicum, Milchzuker, Stärke, Magnesiumstearat, Talk, pflanzliche Oele, Polyalkylenglykole, Vaseline, usw. enthalten. Die pharmazeutischen Präparate können in fester Form, z.B. als Tabletten, Dragées, Suppositorien, Kapseln; oder in flüssiger Form, z.B. als Lösungen, Suspensionen oder Emulsionen, vorliegen. Gegebenenfalls sind sie sterilisiert und bzw. oder enthalten Hilfsstoffe, wie Konservierungs-, Stabiliserungs-, Netz- oder Emulgiermittel, Salze zur Veränderung des osmotischen Druckes, Anaestetica oder Puffer. Sie können auch noch andere therapeutisch wertvolle Stoffe enthalten. Die Verbindungen der Formel I und ihre Salze bzw. Hydrate kommen vorzugsweise für die parenterale Applikation in Betracht und werden zu diesem Zweck bevorzugt als Lyophilisate oder Trockenpulver zur Verdünnung mit üblichen Agenzien wie Wasser oder isotonische Kochsalzlösung, zubereitet. Die leicht hydrolysierbaren Ester bzw. Aether der Verbindungen der Formel I und ihre Salze bzw. Hydrate kommen auch für die enterale Verabreichung in Betracht.

Beispiel 1

Herstellung des Dinatriumsalzes der (6R, 7R) - 7 - [2 - (2 - Amino - 4 - thiazolyl) - 2 - (Z - methoxyimino)acetamido] - 3 - /[2,5 - dihydro - 6 - hydroxy - 2 - methyl - 5 - oxo - as - triazin - 3 - yl)thio]-methyl - 8 - oxo - 5 - thia - 1 - azabicyclo[4.2.0]oct - 2 - en - 2 - carbonsäure.

15,3 g (6R, 7R) - 7 - /2 - [2 - (2 - Chloracetamido) - 4 - thiazolyl] - 2 - (Z - methoxyimino) - acetamido/ - 3 - /[(2,5 - dihydro - 6 - hydroxy - 2 - methyl - 5 - oxo - as - triazin - 3 - yl) - thio]methyl/ - 8 - oxo - 5 - thia - 1 - azabicyclo[4.2.0]oct - 2 - en - 2 - carbonsäure (Fraktion I, siehe nachstehend) werden zusammen mit 5 g Thioharnstoff in 150 ml Wasser suspendiert. Unter guter Stickstoff-Begasung und Rühren wird das pH mit gesättigter Natriumhydrogencarbonatlösung auf 6,8—7,0 gestellt, wobei eine orangefarbige Lösung entsteht. Mittels Autotitrator unter Zugabe von Natriumhydrogencarbonatlösung wird das pH der Reaktionslösung konstant bei 6,8 während 6 Stunden gehalten. Danach werden noch 2,5 g Thioharnstoff hinzugegeben und die Lösung wird weitere 3 Stunden gerührt, wobei das pH unter Zugabe von gesättigter Natriumhydrogencarbonatlösung bei 6,8 gehalten wird. Danach wird die rote Lösung über Nacht im Kühlschrank aufbewahrt, wobei diese dunkler wird. Das pH dieser Lösung wird durch Zugabe von 100%iger Ameisensäure auf 2,0—2,5 eingestellt, wobei die Substanz ausfällt. Diese wird abgenutscht und mit 100 ml 10%iger Ameisensäure gewaschen. Die Mutterlauge wird verworfen. Das bräunliche Nutschgut wird in 200 ml Wasser suspendiert und das pH mit Triäthylamin auf 7 gestellt, wobei eine braune Lösung entsteht. Diese Lösung wird mit 2 g Aktivkohle 30 Minuten gerührt, von der Kohle abfiltriert und das immer noch braune Filtrat mit 100%iger Ameisensäure unter gutem Rühren auf pH 3,5 gestellt. Die habei ausgefallene Substanz wird abgenutscht, mit 50 ml 10%iger Ameisensäure gewaschen und verworfen. Das dunkelgelbe Filtrat wird mit 100%iger

Ameisensäure auf pH 2—2,5 gestellt, wobei die Substanz ausfällt. Diese wird abgenutscht, mit Eiswasser gewaschen und getrocknet. Die erhaltene Cephalosporin-Säure wird, zwecks Ueberführung in das Dinatriumsalz, in einem Gemisch von 40 ml Aceton und 40 ml Wasser suspendiert und mit 20 ml einer 2-n Lösung von 2-Aethylcapronsäure-Natriumsalz in Essigester versetzt. Zur dabei entstandenen orangefarbigen Lösung werden 50 ml Aceton gegeben, wobei ein braunes Harz ausfällt, das mittels Filtration abgetrennt wird. Das gelbe Filtrat wird 30 Minuten gerührt, wobei das Dinatriumsalz kristallisiert. Das Gemisch wird noch portionenweise mit 50 ml Aceton versetzt und über Nacht im Kühlschrank aufbewahrt. Das Kristallisat wird abgenutscht, nacheinander mit einem Aceton-Wasser-Gemisch (85:15), reinem Aceton und tiefsiedendem Petroläther gewaschen und über Nacht im Vakuum bei 40°C getrocknet. Man erhält die Titelsubstanz als beige Kristalle. $[\alpha]_D^{20} = -144°$ (c = 0,5 in Wasser). Das Kernresonanzspektrum und die Mikroanalyse entsprechen der angegebenen Struktur.

Die als Ausgangsprodukt eingesetzte (6R, 7R) - 7 - /2 - [2 - (2 - Chloracetamido) - 4 - thiazolyl] - 2 - (Z - methoxyimino)acetamido/ - 3 - /[(2,5 - dihydro - 6 - hydroxy - 2 - methyl - 5 - oxo - as - triazin - 3 - yl)thio]methyl/ - 8 - oxo - 5 - thia - 1 - azabicyclo[4.2.0]oct - 2 - en - 2 - carbonsäure kann wie folgt hergestellt werden:

22,24 g 2-(2-Chloracetamido-thiazol-4-yl)-2-(Z-methoxyimino)-essigsäure werden in 240 ml Methylenchlorid suspendiert. Dieser Suspension werden 13,39 ml Triäthylamin zugegeben, wobei eine hellbraune Lösung entsteht. Diese Lösung wird auf 0—5°C gekühlt und mit 16,72 g Phosphorpentachlorid versetzt, 5 Minuten bei 0—5°C und 20 Minuten ohne Kühlung gerührt. Die gelbe Lösung wird am Vakuum bei 35°C eingedampft. Der Eindampfrückstand wird zweimal mit n-Heptan geschüttelt und letzteres abdekantiert. Der harzige Rückstand wird mit 240 ml Tetrahydrofuran behandelt und das ungelöste Triäthylamin-hydrochlorid wird abfiltriert. Das gelbe Filtrat enthält das Säurechlorid.

22 g (7R)-7-Amino-3-desacetoxy-3-[(2,5-dihydro-6-hydroxy-2-methyl-5-oxo-as-triazin-3-yl)-thio]-cephalosporansäure werden in einem Gemisch von 300 ml Wasser und 150 ml Tetrahydrofuran suspendiert. Zur Suspension wird unter guter Stickstoff-Begasung mit Hilfe des Autotitrators 2-n Natronlauge zugetropft, bis eine braunrote Lösung von pH 8 entsteht. Diese wird auf 0—5°C gekühlt und während 15 Minuten tropfenweise mit der oben hergestellten Lösung des Säurechlorids in Tetrahydrofuran versetzt. Danach wird 2 1/2 Stunden bei 25°C gerührt. Das pH des Acylierungsgemisches wird unter Zugabe von 2-n Natronlauge konstant bei 8 gehalten. Die praktisch schwarze Lösung wird am Vakuum bei 40°C von Tetrahydrofuran befreit. Nun werden 100 ml 2-n Schwefelsäure zugegeben. Die dabei ausgefallene Substanz wird abgenutscht, mit Wasser gewaschen und gut abgesaugt. Das feuchte, braune Nutschgut wird in 1,5 1 Aceton gelöst. Die dunkle Lösung wird von wenig dunklem ungelöstem Material über Hyflo abfiltriert, mit Kohle versetzt, 30 Minuten gerührt und wieder über Hyflo filtriert. Das orange-rote Filtrat wird über Natriumsulfat getrocknet, am Vakuum eingeengt und mit Essigester abgedampft. Dabei fällt ein schwarzes Harz aus, das abfiltriert und verworfen wird. Das 2-phasige, noch Wasser enthaltende Filtrat wird dreimal mit Benzol am Vakuum bei 40°C azeotropiert. Die dabei ausgefallene Substanz wird abgenutscht und im Vakuum bei 40°C getrocknet. Letztere wird zweimal mit je 1 1 Aceton verrührt, wobei ein braunes Harz zurückbleibt, das verworfen wird. Die vereinigten orangefarbigen Acetonextrakte werden am Vakuum bei 40°C auf ca. 150 ml eingeengt, wobei ein braunes Harz abfiltriert und verworfen wird. Das Filtrat wird mit 1 Liter Essigester versetzt und am Vakuum bei 40°C eingeengt. Die dabei ausgefallene Substanz wird abgenutscht, mit Essigester und danach mit Aether gewaschen [(6R, 7R)-7-/2-[2-(2-Chloracetamido)-4-thiazolyl]-2-(Z-methoxyimino)acetamido/-3-/[(2,5-dihydro-6-hydroxy-2-methyl-5-oxo-as-triazin-3-yl)thio]-methyl - 8 - oxo-5-thia-1-azabicyclo[4.2.0]oct-2-en-2-carbonsäure, Fraktion 1: eine beige, amorphe Säure]. Diese Fraktion I kann direkt für die Herstellung des gewünschten Endproduktes eintgesetzt werden.

Die Essigestermutterlauge wird am Vakuum bei 40°C stark eingengt, mit Aether verdünnt und die ausgefallene Substanz abgenutscht [(6R, 7R) - 7 - /2 - [2 - (2 - Chloracetamido) - 4 - thiazolyl] - 2 - (Z - methoxyimino)acetamido/ - 3 - /[(2,5 - dihydro - 6 - hydroxy - 2 - methyl - 5 - oxo - as - triazin - 3 - yl)thio] - methyl/ - 8 - oxo - 5 - thia - 1 - azabicyclo[4.2.0]oct - 2 - en - 2 - carbonsäure, Fraktion II: eine hellbeige amorphe Säure, dünnschichtchromatographisch etwas reiner als Fraktion I].

Zur Herstellung des Dinatriumsalzes werden 3,5 g Säure (Fraktion II) in einem Gemisch von 20 ml Aceton und 11 ml Wasser gelöst. Die Lösung wird mit 7 ml einer 2-n Lösung von 2-Aethylcapronsäure-Natriumsalz in Essigester versetzt, wobei das Dinatriumsalz kristallisiert. Nun werden noch portionenweise 25 ml Aceton hinzugefügt und das Gemisch 2 Stunden im Tiefkühlschrank aufbewahrt. Danach wird das Kristallisat abgenutscht, nacheinander mit 25 ml eines eiskalten Aceton-Wasser-Gemisches (80:20), reinem Aceton und tiefsiedendem Petroläther gewaschen und über Nacht im Hochvakuum bei 40°C getrocknet. Man erhält das Dinatriumsalz der (6R, 7R) - 7 - /2 - [2 - (2 - Chloroacetamido) - 4 - thiazolyl] - 2 - (Z - methoxyimino)acetamido/ - 3 - /[(2,5 - dihydro -6 hydroxy - 2 - methyl - 5 - oxo - as - triazin - 3 - yl)thio] - methyl/ -8 - oxo - 5 - thia -1 azabicyclo[4.2.0]oct - 2 - en - 2 - carbonsäure als hellgelbe Kristalle. $[\alpha]_D^{20} = -142,7°$ (c = 1 in Wasser). Das Kernresonanzspektrum und die Mikroanalyse entsprechen der angegebenen Struktur.

Beispiel 2

Herstellung von Methylen - (6R, 7R) - 7 - [2 - (2 - Amino - 4 - thiazolyl) - 2 - (Z - methoxyimino)acetamido] -3 [[[2,5 - dihydro - 2 - methyl - 5 - oxo - 6 - [(pivaloyloxy)methoxy] - as - triazin - 3 - yl]thio]methyl] - 8 - oxo - 5 - thia - 1 - azabicyclo[4.2.0]oct - 2 - en - 2 - carboxylat - pivalat.

1,85 g des nach Beispiel 1 hergestellten Cephalosporin-Dinatriumsalzes werden in 50 ml Dimethylformamid suspendiert und unter Stickstoff-Begasung bei 0—5°C mit 1,35 g Pivaloyloxymethyljodid versetzt. Das Reaktionsgemisch wird 30 Minuten bei 0—5°C gerührt und danach auf 500 ml Essigester gegossen. Das Gemisch wird 3 mal mit Wasser, 2 mal mit 5%iger Natriumhydrogencarbonatlösung und schliesslich nochmals mit Wasser gewaschen. Die Lösung wird über Natriumsulfat getrocknet und am Vakuum bei 35°C stark eingeengt. Nach Zugabe von Aether fällt die Titelsubstanz amorph aus. Diese wird abgenutscht, mit Aether und tiefsiedendem Petroläther gewaschen und über Nacht im Hochvakuum bei 25°C getrocknet. Man erhält die Titelsubstanz als beiges amorphes Pulver. Das Kernresonanzspektrum und die Mikroanalyse sind mit der angegebenen Struktur im Einklang.

Beispiel 3

Herstellung von Trockenampullen für die intramuskuläre Verabreichung:

Es wird in üblicher Weise ein Lyophilisat von 1 g des Dinatriumsalzes der (6R, 7R) - 7 - [2 - (2 - Amino - 4 - thiazolyl) - 2 - (Z - methoxyimino)acetamido] - 3 - /[(2,5 - dihydro - 6 - hydroxy - 2 - methyl - 5 - oxo - as - triazin - 3 - yl)thio]methyl/ - 8 - oxo - 5 - thia - 1 - azabicyclo[4.2.0]oct - 2 - en - 2 - carbonsäure hergestellt und in eine Ampulle abgefüllt. Vor der Verabreichung wird das Lyophilisat mit 2,5 ml einer 2%igen wässrigen Lidocainhydrochlorid-Lösung versetzt.

Beispiel 4

Es wird in üblicher Weise eine Gelatine-Steckkapsel folgender Zusammensetzung hergestellt:

| | |
|---|---|
| Methylen (6R, 7R)-7-[2-(2-amino-4-thiazolyl)-2-(Z-methoxyimino)acetamido]-3-[[[2,5-dihydro-2-methyl-5-oxo-6-[(pivaloyloxy)methoxy]-as-triazin-3-yl]thio]methyl]-8-oxo-5-thia-1-azabicyclo[4.2.0]oct-2-en-2-carboxylat-pivalat | 500 mg |
| Luviskol (wasserlösliches Polyvinylpyrrolidon) | 20 mg |
| Mannit | 20 mg |
| Talk | 15 mg |
| Magnesiumstearat | 2 mg |
| | 557 mg |

**Patentansprüche für die Vertragsstaaten: BE CH DE FR IT LU NL SE**

1. Cephalosporinderivat der allgemeinen Formel

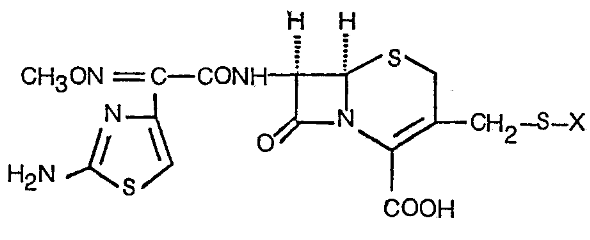

I

in der X die 1,2,5,6-Tetrahydro-2-methyl-5,6-dioxo-as-triazin-3-ylgruppe bzw. die 2,5-Dihydro-6-hydroxy-2-methyl-5-oxo-as-triazin-3-ylgruppe bedeutet, sowie leicht hydrolysierbare Ester/Aether und Salze dieser Verbindung und Hydrate der Verbindung der Formel I bzw. von deren Estern/Aethern und Salzen.

2. Cephalosporinderivat nach Anspruch 1 in der synisomeren Form bzw. Gemische, in denen die syn-isomere Form überwiegt.

3. (6R, 7R) - 7 - [2 - (2 - Amino - 4 - thiazolyl) - 2 - (Z - methoxyimino)acetamido] - 3 - /[2,5 - di-.hydro - 6 - hydroxy - 2 - methyl - 5 - oxo - as - triazin - 3 - yl)thio] - methyl/ - 8 - oxo - 5 - thia - 1 - azabicyclo[4.2.0]oct - 2 - en - 2 - carbonsäure sowie Salze dieser Verbindung und Hydrate dieser Verbindung bzw. Salze.

4. Methylen (6R, 7R) - 7 - [2 - (2 - amino - 4 - thiazolyl) - 2 - (Z - methoxyimino)acetamido] - 3 - [[[2,5 - dihydro - 2 - methyl - 5 - oxo - 6 - [(pivaloyloxy)methoxy] - as - triazin - 3 - yl]thio]methyl] - 8 - oxo - 5 - thia - 1 - azabicyclo[4.2.0]oct - 2 - en - 2 - carboxylatpivalat sowie Salze dieser Verbindung und Hydrate dieser Verbindung und Salze.

5. Verbindungen gemäss einem der Ansprüche 1—4 als pharmazeutische Wirkstoffe zur Behandlung und Prophylaxe von Infektionskrankheiten.

6. (6R, 7R) - 7 - [2 - (2 - Amino - 4 - thiazolyl) - 2 - (Z - methoxyimino)acetamido] - 3 - /[2,5 - dihydro - 6 - hydroxy - 2 - methyl - 5 - oxo - as - triazin - 3 - yl)thio] - methyl/ - 8 - oxo - 5 - thia - 1 - azabicyclo[4.2.0]oct - 2 - en - 2 - carbonsäure sowie Salze dieser Verbindung und Hydrate dieser Verbindung bzw. Salze als pharmazeutische Wirkstoffe zur Behandlung und Prophylaxe von Infektionskrankheiten.

7. Pharmazeutische Präparate, gekennzeichnet durch einen Gehalt an einer Verbindung der allgemeinen Formel

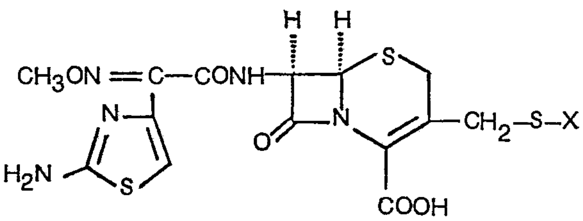

I

in der X die 1,2,5,6-Tetrahydro-2-methyl-5,6-dioxo-as-triazin-3-ylgruppe bzw. die 2,5-Dihydro-6-hydroxy-2-methyl-5-oxo-as-triazin-3-ylgruppe bedeutet, oder an einem leicht hydrolysierbaren Ester/Aether an einem Salz einer solchen Verbindung oder an einem Hydrat einer Verbindung der Formel I bzw. eines Esters/Aethers oder Salzes davon, in Verbindung mit einem pharmazeutischen Trägermaterial.

8. Präparat gemäss Anspruch 7, dadurch gekennzeichnet, dass es (6R, 7R)-7-[2-(2-Amino-4-thiazolyl) - 2 - (Z - methoxyimino)acetamido] - 3 - /[(2,5 - dihydro - 6 - hydroxy - 2 - methyl - 5 - oxo - as - triazin - 3 - yl)thio] - methyl/ - 8 - oxo - 5 - thia - 1 - azabicyclo[4.2.0]oct - 2 - en - 2 - carbonsäure, ein Salz dieser Verbindung oder ein Hydrat dieser Verbindung bzw. dieses Salzes enthält.

9. Pharmazeutische Präparate zur Behandlung und Prophylaxe von Infektionskrankheiten gekennzeichnet durch einen Gehalt an einer Verbindung der allgemeinen Formel

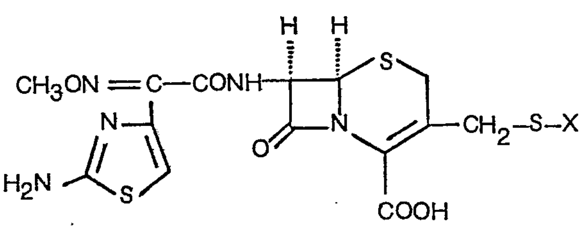

I

in der X die 1,2,5,6-Tetrahydro-2-methyl-5,6-dioxo-as-triazin-3-ylgruppe bzw. die 2,5-Dihydro-6-hydroxy-2-methyl-5-oxo-as-triazin-3-ylgruppe bedeutet, oder an einem leicht hydrolysierbaren Ester/Aether, an einem Salz einer solchen Verbindung oder an einem Hydrat einer Verbindung der Formel I bzw. eines Esters/Aethers oder Salzes davon, in Verbindung mit einem pharmazeutischen Trägermaterial.

10. Präparat gemäss Anspruch 9, dadurch gekennzeichnet, das es (6R, 7R)-7-[2-(2-Amino-4-thiazolyl) - 2 - (Z - methoxyimino)acetamido] - 3 - /[(2,5 - dihydro - 6 - hydroxy - 2 - methyl - 5 - oxo - as - triazin - 3 - yl)thio] - methyl/ - 8 - oxo - 5 - thia - 1 - azabicyclo[4.2.0]oct - 2 - en - 2 - carbonsäure, ein Salz dieser Verbindung oder ein Hydrat dieser Verbindung bzw. dieses Salzes enthält.

11. Verfahren zur Herstellung der Verbindungen gemäss einem der Ansprüche 1—4 dadurch gekennzeichnet, dass man

a) in einer Verbindung der allgemeinen Formel

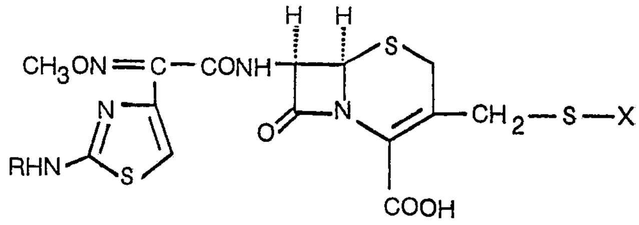

II

in der X die in Anspruch 1 gegebene Bedeutung hat, R eine abspaltbare Schutzgruppe darstellt und die Carboxygruppe in geschützter Form vorliegen kann, die Schutzgruppe R, ggfs. auch eine allenfalls vorhandene Carboxyschutzgruppe, abspaltet oder dass man

b) zur Herstellung eines leicht hydrolysierbaren Esters/Aethers der Verbindung der Formel I diese einer entsprechenden Veresterung bzw. Verätherung unterwirft, oder dass man

c) zur Herstellung von Salzen oder Hydraten der Verbindung der Formel I bzw. Hydraten dieser Salze die Verbindung der Formel I in ein Salz oder Hydrat bzw. ein Hydrat dieses Salzes überführt.

**Patentansprüche für den Vertragsstaat: AT**

1. Verfahren zur Herstellung eines Cephalosporinderivats der allgemeinen Formel

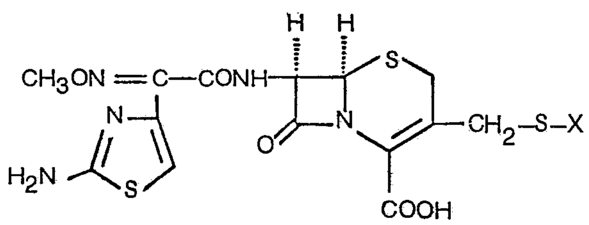

I

in der X die 1,2,5,6-Tetrahydro-2-methyl-5,6-dioxo-as-triazin-3-ylgruppe bzw. die 2,5-Dihydro-6-hydroxy-2-methyl-5-oxo-as-triazin-3-ylgruppe bedeutet, sowie von leicht hydrolysierbaren Estern/Aethern und Salzen dieser Verbindung sowie von Hydraten der Verbindung der Formel I bzw. von deren Estern/Aethern und Salzen, dadurch gekennzeichnet, dass man

a) in einer Verbindung der allgemeinen Formel

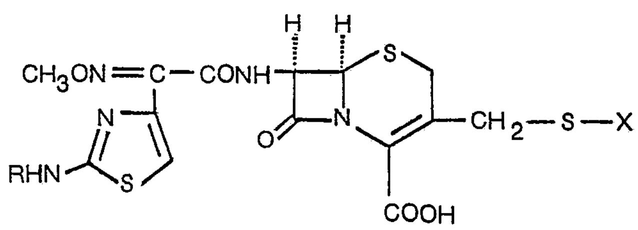

II

in der X die oben gegebene Bedeutung hat, R eine abspaltbare Schutzgruppe darstellt und die Carboxygruppe in geschützter Form vorliegen kann, die Schutzgruppe R, gegebenenfalls auch eine allenfalls vorhandene Carboxyschutzgruppe, abspaltet oder dass man

b) zur Herstellung eines leicht hydrolysierbaren Esters/Aethers der Verbindung der Formel I diese einer entsprechenden Veresterung bzw. Verätherung unterwirft, oder dass man

c) zur Herstellung von Salzen oder Hydraten der Verbindung der Formel I bzw. Hydraten dieser Salze die Verbindung der Formel I in ein Salz oder Hydrat bzw. ein Hydrat dieses salzes überführt.

2. Verfahren nach Anspruch 1, dadurch gekennzeichnet, dass man eine Ausgangsverbindung der Formel II, worin R Chloracetyl darstellt, mit Thioharnstoff behandelt.

3. Verfahren nach Anspruch 1 oder 2 zur Herstellung einer Verbindung der Formel I in der synisomeren Form bzw. von Gemischen, in denen die syn-isomere Form überwiegt, dadurch gekennzeichent, dass man eine Ausgangsverbindung der Formel II mit dieser Konfiguration einsetzt oder ein erhaltenes syn/anti-Gemisch in üblicher Weise auftrennt.

4. Verfahren nach einem der Ansprüche 1—3 zur Herstellung von (6R, 7R)-7-[2-(2-Amino-4-thiazolyl) - 2 - (Z - methoxyimino)acetamido] - 3 - /[(2,5 - dihydro - 6 - hydroxy - 2 - methyl - 5 - oxo - as - triazin - 3 - yl)thio] - methyl/ - 8 - oxo - 5 - thia - 1 - azabicyclo[4.2.0]oct - 2 - en - 2 - carbonsäure sowie von Salzen dieser Verbindung und von Hydraten dieser Verbindung bzw. Salze, dadurch gekennzeichnet, dass man entsprechend substituierte Ausgangsverbindungen einsetzt.

5. Verfahren nach einem der Ansprüche 1—3 zur Herstellung von Methylen (6R, 7R)-7-[2-(2-amino - 4 - thiazolyl) - 2 - (Z - methoxyimino)acetamido] - 3 - /[(2,5 - dihydro - 2 - methyl - 5 - oxo - 6 - [(pivaloyloxy)methoxy] - as - triazin - 3 - yl)thio] - methyl/ - 8 - oxo - 6 - thia - 1 - azabicyclo[4.2.0]oct - 2 - en - 2 - carboxylat - pivalat sowie von Salzen dieses Esters und von Hydraten dieses Esters bzw. dieser Salze, dadurch gekennzeichnet, dass man (6R, 7R) - 7 - [2 - (2 - Amino - 4 - thiazolyl) - 2 - (Z - methoxyimino)acetamido] - 3 - /[(2,5 - dihydro - 6 - hydroxy - 2 - methyl - 5 - oxo - as - triazin - 3 - yl)thio] - methyl/ - 8 - oxo - 5 - thia - 1 - azabicyclo[4.2.0]oct - 2 - en - 2 - carbonsäure, vorzugsweise in Form des Dinatriumsalzes, mit einem Pivaloyloxymethylhalogenid, vorzugsweise mit dem Jodid, umsetzt und erwünschtenfalls das erhaltene Produkt in ein Salz oder Hydrat bzw. ein Hydrat dieses Salzes überführt.

**Claims for the Contracting States: BE CH DE FR IT LU NL SE**

1. Cephalosporin derivatives of the general formula

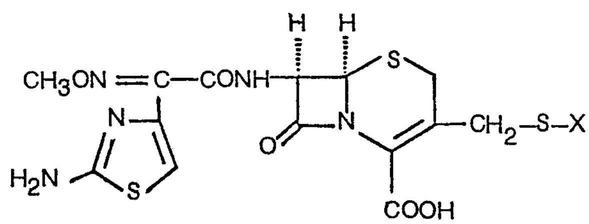

I

in which X signifies the 1,2,5,6-tetrahydro-2-methyl-5,6-dioxo-as-triazin-3-yl group or the 2,5-dihydro-6-hydroxy-2-methyl-5-oxo-as-triazin-3-yl group, as well as readily hydrolyzable esters/ethers and salts of this compound and hydrates of the compound of formula I or of its esters/ethers and salts.

2. Cephalosporin derivative according to claim 1 in the syn-isomeric form or mixtures in which the syn-isomeric form predominates.

3. (6R, 7R) - 7 - [2 - (2 - Amino - 4 - thiazolyl) - 2 - (Z - methoxyimino)acetamido) - 3 - /[2,5 - dihydro - 6 - hydroxy - 2 - methyl - 5 - oxo - as - triazin - 3 - yl)thio] - methyl/ - 8 - oxo - 5 - thia - 1 - azabicyclo[4.2.0]oct - 2 - ene - 2 - carboxylic acid as well as salts of this compound and hydrates of this compound or salts.

4. Methylene (6R, 7R) - 7 - [2 - amino - 4 - thiazolyl) - 2 - (Z - methoxyimino)acetamido] - 3 - [[[2,5 - dihydro - 2 - methyl - 5 - oxo - 6 - [(pivaloyloxy)methoxy] - as - triazin - 3 - yl]thio]methyl] - 8 - oxo - 5 - thia - 1 - azabicyclo[4.2.0]oct - 2 - ene - 2 - carboxylate pivalate as well as salts of this compound and hydrates of this compound and salts.

5. Compounds in accordance with one of claims 1—4 as pharmaceutically active substances for the treatment and prophylaxis of infectious diseases.

6. (6R, 7R) - 7 - [2 - (2 - Amino - 4 - thiazolyl) - 2 - (Z - methoxyimino)acetamido] - 3 - /[2,5 - dihydro - 6 - hydroxy - 2 - methyl - 5 - oxo - as - triazin - 3 - yl)thio] - methyl/ - 8 - oxo - 5 - thia - 1 - azabicyclo[4.2.0]oct - 2 - ene - 2 - carboxylic acid as well as salts of this compound and hydrates of this compound or salts as pharmaceutically active substances for the treatment and prophylaxis of infectious diseases.

7. Pharmaceutical preparations, characterized by a content of a compound of the general formula

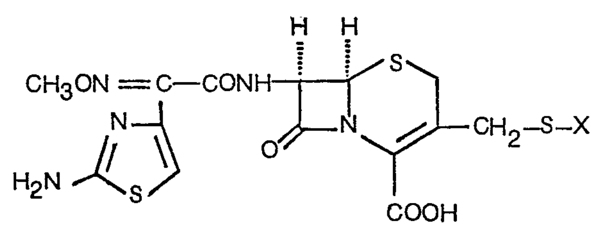

I

in which X signifies the 1,2,5,6-tetrahydro-2-methyl-5,6-dioxo-as-triazin-3-yl group or the 2,5-dihydro-6-hydroxy-2-methyl-5-oxo-as-triazin-3-yl group, or of a readily hydrolyzable ester/ether, of a salt of such a compound or of a hydrate of a compound of formula I or of an ester/ether or salt thereof, in combination with a pharmaceutical carrier material.

8. Preparation in accordance with claim 7, characterized in that it contains (6R, 7R)-7-[2-(2-amino - 4 - thiazolyl) - 2 - (Z - methoxyimino)acetamido] - 3 - /[(2,5 - dihydro - 6 - hydroxy - 2 - methyl - 5 - oxo - as - triazin - 3 - yl)thio] - methyl/ - 8 - oxo - 5 - thia - 1 - azabicyclo[4.2.0]oct - 2 - ene - 2 - carboxylic acid, a salt of this compound or a hydrate of this compound or of this salt.

9. Pharmaceutical preparations for the treatment and prophylaxis of infectious diseases, characterized by a content of a compound of the general formula

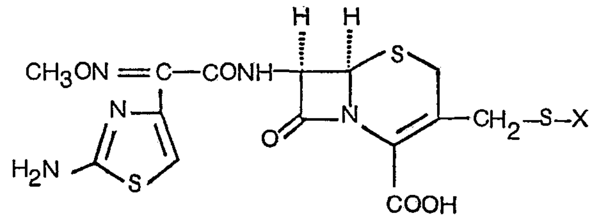

I

in which X signifies the 1,2,5,6-tetrahydro-2-methyl-5,6-dioxo-as-triazin-3-yl group or the 2,5-dihydro-6-hydroxy-2-methyl-5-oxo-as-triazin-3-yl group, or of a readily hydrolyzable ester/ether, of a salt of such a compound or of a hydrate of a compound of formula I or of an ester/ether or salt thereof, in combination with a pharmaceutical carrier material.

10. Preparation in accordance with claim 9, characterized in that it contains (6R, 7R)-7-[2-(2-amino - 4 - thiazolyl) - 2 - (Z - methoxyimino)acetamido] - 3 - /[(2,5 - dihydro - 6 - hydroxy - 2 - methyl - 5 - oxo - as - triazin - 3 - yl)thio] - methyl/ - 8 - oxo - 5 - thia - 1 - azabicyclo[4.2.0]oct - 2 - ene - 2 - carboxylic acid, a salt of this compound or a hydrate of this compound or of this salt.

11. Process for the manufacture of the compounds in accordance with one of claims 1—4, characterized by

a) cleaving off the protecting group R, and, if desired, also a carboxy protecting group which may be present, in a compound of the general formula

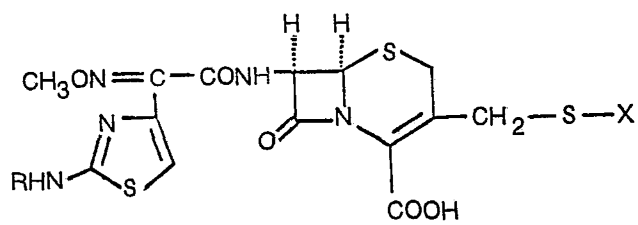

II

in which X has the significance given in claim 1, R represents a cleavable protecting group and the carboxy group can be present in protected form, or by

b) for the manufacture of a readily hydrolyzable ester/ether of the compound of formula I, subjecting this to a corresponding esterification or etherification, or by

c) for the manufacture of salts or hydrates of the compound of formula I or hydrates of these salts, converting the compound of formula I into a salt or hydrate or into a hydrate of this salt.

**Claims for the Contracting State: AT**

1. Process for the manufacture of a cephalosporin derivative of the general formula

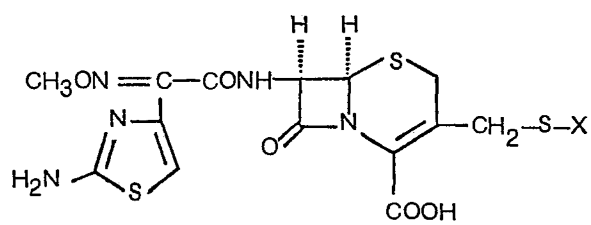

I

in which X signifies the 1,2,5,6-tetrahydro-2-methyl-5,6-dioxo-as-triazin-3-yl group or the 2,5-dihydro-6-hydroxy-2-methyl-5-oxo-as-triazin-3-yl group, as well as of readily hydrolyzable esters/ethers and salts of this compound as well as of hydrates of the compound of formula I or of its esters/ethers and salts, characterized by

a) cleaving off the protecting group R, and, if desired, also a carboxy protecting group which may be present, in a compound of the general formula

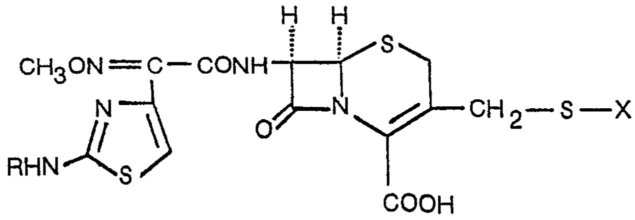

II

in which X has the significance given above, R represents a cleavable protecting group and the carboxy group can be present in protected form, or by

b) for the manufacture of a readily hydrolyzable ester/ether of the compound of formula I, subjecting this to a corresponding esterification or etherification, or by

c) for the manufacture of salts or hydrates of the compound of formula I or hydrates of these salts, converting the compound of formula I into a salt or hydrate or into a hydrate of this salt.

2. Process according to claim 1, characterized in that a starting compound of formula II, wherein R represents chloroacetyl, is treated with thiourea.

3. Process according to claim 1 or 2 for the manufacture of a compound of formula I in the syn-isomeric form or of mixtures in which the syn-isomeric form predominates, characterized by using a starting compound of formula II with this configuration or separating an obtained syn/anti mixture in the usual manner.

4. Process according to one of claims 1—3 for the manufacture of (6R, 7R)-7-[2-(2-amino-4-thiazolyl) - 2 - (Z - methoxyimino)acetamido] - 3 - /[(2,5 - dihydro - 6 - hydroxy - 2 - methyl - 5 - oxo - as - triazin - 3 - yl-thio] - methyl/ - 8 - oxo - 5 - thia - 1 - azabicyclo[4.2.0]oct - 2 - ene - 2 - carboxylic acid as well as of salts of this compound and of hydrates of this compound or salts, characterized by using corresponding substituted starting compounds.

5. Process according to one of claims 1—3 for the manufacture of methylene (6R, 7R)-7-[2-(2-amino - 4 - thiazolyl) - 2 - (Z - methoxyimino)acetamido] - 3 - /[(2,5 - dihydro - 2 - methyl - 5 - oxo - 6 - [(pivaloyloxy)methoxy] - as - triazin - 3 - yl)thio] - methyl/ - 8 - oxo - 6 - thia - 1 - azabicyclo[4.2.0]oct - 2 - ene - 2 - carboxylate pivalate as well as of salts of this ester and of hydrates of this ester or of these salts, characterized by reacting (6R, 7R) - 7 - [2 - (2 - amino - 4 - thiazolyl) - 2 - (Z - methoxyimino) - acetamido] - 3 - /[(2,5 - dihydro - 6 - hydroxy - 2 - methyl - 5 - oxo - as - triazin - 3 - yl)thio] - methyl/ - 8 - oxo - 5 - thia - 1 - azabicyclo[4.2.0]oct - 2 - ene - 2 - carboxylic acid, preferably in the form of the disodium salt, with a pivaloyloxymethyl halide, preferably with the iodide, and, if desired, converting the product obtained into a salt or hydrate or into a hydrate of this salt.

**Revendications pour les Etats contractants: BE CH DE FR IT LU NL SE**

1. Dérivés de céphalosporine de formule générale

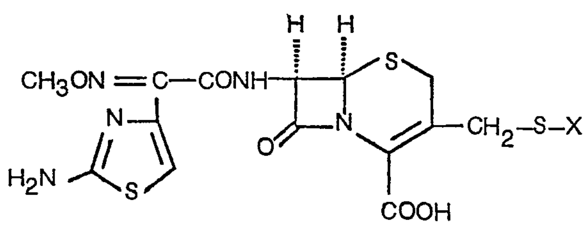

I

où X représente le groupe 1,2,5,6-tétrahydro-2-méthyl-5,6-dioxo-as-triazin-3-yle ou le groupe 2,5-dihydro-6-hydroxy-2-méthyl-5-oxo-as-triazin-3-yle, ainsi que les esters/éthers et sels facilement hydrolysables de ce composé et les hydrates du composé de formule I ou de ses esters/éthers et sels.

2. Dérivé de céphalosporine selon la revendication 1 sous la forme isomère syn ou mélanges où cette forme isomérique syn prédomine.

3. Acide (6R, 7R) - 7 - [2 - (2 - amino - 4 - thiazolyl) - 2 - (Z - méthoxyimino) - acetamido] - 3 - /[(2,5 - dihydro - 6 - hydroxy - 2 - méthyl - 5 - oxo - as - triazin - 3 - yl)thio] - méthyl/ - 8 - oxo - 5 - thia - 1 - azabicyclo[4.2.0]oct - 2 - ène - 2 - carboxylique ainsi que les sels de ce composé et les hydrates de ce composé ou leurs sels.

4. Méthylène (6R, 7R) - 7 - [2 - (2 - amino - 4 - thiazolyl) - 2 - (Z - méthoxyimino)acétamido] - 3 - ] - 3 - [[[2,5 - dihydro - 2 - méthyl - 5 - oxo - 6 - [(pivaloyloxy)méthoxy] - as - triazin - 3 - yl]thio] - méthyl] - 8 - oxo - 5 - thia - 1 - azabicyclo[4.2.0]oct - 2 - èn - 2 - carboxylate - pivalate ainsi que les sels de ce composé et les hydrates de ce composé et leurs sels.

5. Composés selon l'une des revendications 1—4 comme substances pharmaceutiques actives pour le traitement et la prophylaxie des maladies infectieuses.

6. Acide (6R, 7R)-7-[2-Amino-4-thiazolyl)-2-(Z-méthoxyimino)-acétamido]-3-/[2,5-dihydro-6-hydroxy - 2 - méthyl - 5 - oxo - as - triazin - 1 - yl)thio] - méthyl/ - 8 - oxo - 5 - thia - 1 - azabicyclo[4.2.0]oct - 2 - èn - 2 - carboxylique ainsi que les sels de ce composé et les hydrates de ce composé ou leurs sels comme substances actives pharmaceutiques aux fins de traitement et de prophylaxie de maladies infectieuses.

7. Préparations pharmaceutiques, caractérisées en ce qu'elles contiennent un composé de formule générale

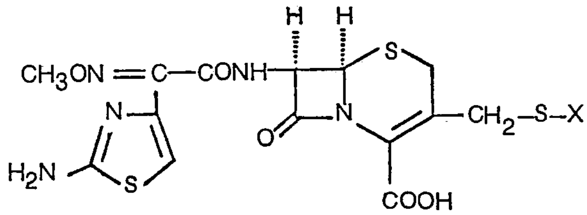

I

où X représente le groupe 1,2,5,6-tétrahydro-2-méthyl-5,6-dioxo-as-triazin-3-yle ou le groupe 2,5-dihydro-6-hydroxy-2-méthyl-5-oxo-as-triazin-3-yle, ou un ester/éther facilement hydrolysable, un sel d'un tel composé ou un hydrate d'un composé de formula I ou d'un ester/éther ou d'un sel de ce corps, en liaison avec un support pharmaceutique.

8. Préparation selon la revendication 7, caractérisée en ce qu'elle contient l'acide (6R, 7R)-7-[2-(2-Amino-4-thiazolyl)-2-(Z-méthoxyimino)-acétamido]-3-/[(2,5-dihydro-6-hydroxy-2-méthyl-5-oxo-as-triazin-3-yl)thio]-méthyl/-8-oxo-5-thia-1-azabicyclo[4.2.0]oct-2-èn-2-carboxylique, un sel de ce composé ou un hydrate de ce composé ou de ce sel.

9. Préparations pharmaceutiques destinées au traitement et à la prophylaxie des maladies infectieuses, caractérisées en ce qu'elles contiennent on composé de formule générale

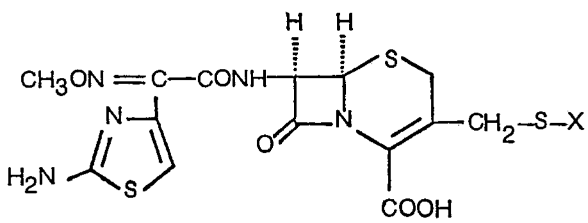

I

où X représente le groupe 1,2,5,6-tétrahydro-2-méthyl-5,6-dioxo-as-triazin-3-yle ou le groupe 2,5-dihydro-6-hydroxy-2-méthyl-5-oxo-as-triazin-3-yle, ou un ester/éther facilement hydrolysable, un sel d'un tel composé ou un hydrate d'un composé de formule I ou d'un ester/éther ou sel de ce corps, en liaison avec un support pharmaceutique.

10. Préparation selon la revendication 9, caractérisée en ce qu'elle contient l'acide (6R, 7R)-7-[2-(2-Amino-4-thiazolyl)-2-(Z-méthoxyimino)-acétamido]-3-/[(2,5-dihydro-6-hydroxy-2-méthyl-5-oxo-as-triazin-3-yl)thio]-méthyl/-8-oxo-5-thia-1-azabicyclo[4.2.0]oct-2-èn-2-carboxylique, un sel de ce composé ou un hydrate de ce composé ou de ce sel.

11. Procédé de préparation des composés selon l'une des revendications 1—4, caractérisé en ce que

a) dans un composé de formule générale

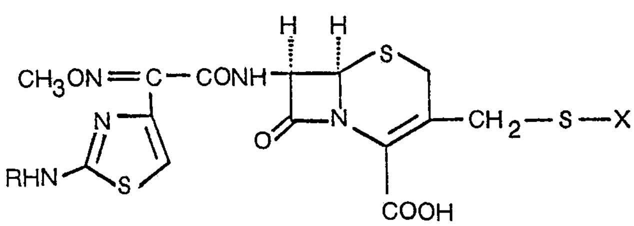

II

où X a la signification donnée dans la revendication 1, R représente un groupe protecteur séparable et le groupe carboxy peut se présenter sous forme protégée, on sépare le groupe protecteur R, ainsi le cas échéant qu'un groupe protecteur carboxy éventuellement présent, ou

b) pour préparer un ester/éther facilement hydrolysable du composé de formule I on soumet celui-ci à une estérification ou éthérification correspondante, ou

c) pour préparer des sels ou des hydrates du composé de formule I ou des hydrates de ces sels, on transforme le composé de formule I en un sel ou un hydrate ou un hydrate de ce sel.

**Revendications pour l'Etat contractant: AT**

1. Procédé de préparation d'un dérivé de céphalosporine de formule générale

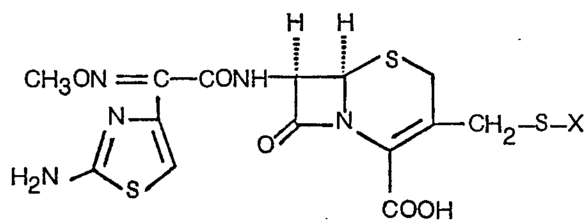

I

où X représente le groupe 1,2,5,6-tétrahydro-2-méthyl-5,6-dioxo-as-triazin-3-yle ou le groupe 2,5-dihydro-6-hydroxy-2-méthyl-5-oxo-as-triazin-3-yle, ainsi que d'esters/éthers facilement hydrolysables et de sels de ce composé, ainsi que d'hydrates du composé de formule I ou de leurs esters/éthers et sels, caractérisé en ce que

a) dans un composé de formule générale

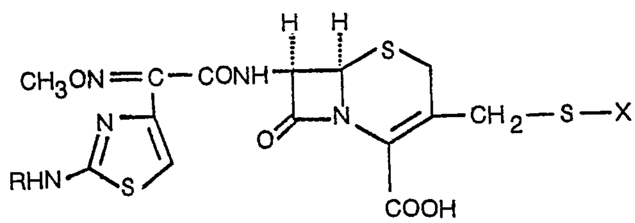

II

où X a la signification donnée ci-dessus, R représente un groupe protecteur séparable et le groupe carboxy peut se présenter sous forme protégée, on sépare le groupe protecteur R, ainsi le cas échéant qu'un groupe protecteur carboxy éventuellement présent, ou

b) pour préparer un ester/éther facilement hydrolysable du composé de formule I, on soumet celui-ci à une estérification ou à une éthérification correspondante, ou

c) pour préparer des sels ou des hydrates du composé de formule I ou des hydrates de ces sels, on transforme le composé de formule I en un sel hydrate ou en un hydrate de ce sel.

2. Procédé selon la revendication 1, caractérisé en ce qu'on traite un composé de départ de formule II, où R représente un chloracétyle, avec de la thiourée.

3. Procédé selon la revendication 1 ou la revendication 2 de préparation d'un composé de formule I sous la forme isomère syn ou sous celle de mélanges où la forme isomérique syn prédomine, caractérisé en ce qu'on utilise un composé de départ de formule II ayant cette configuration ou en ce qu'on dédouble un mélange syn/anti obtenu de manière habituelle.

4. Procédé selon l'une des revendications 1—3 de préparation d'acide (6R, 7R)-7-[2-(2-Amino-4-thiazolyl) - 2 - (Z - méthoxyimino)acétamido] - 3 - /[(2,5 - dihydro - 6 - hydroxy - 3 - méthyl - 5 - oxo - as - triazin - 3 - yl)thio] - méthyl/ - 8 - oxo - 5 - thia - 1 - aza - bicyclo[4.2.0]oct - 2 - èn - 2 - carboxylique ainsi que de sels de ce composé et d'hydrates de ce composé ou de leurs sels, caractérisé en ce qu'on utilise des composés de départ substitués de façon correspondante.

5. Procédé selon l'une des revendications 1—3 de préparation de méthylène (6R, 7R)-7-[2-(2-amino - 4 - thiazolyl) - 2 - (Z - méthoxyimino)acétamido] - 3 - /[(2,5 - dihydro - 2 - méthyl - 5 - oxo - 6 - [(pivaloyloxy)méthoxy] - as - triazin - 8 - yl)thio - méthyl/ - 8 - oxo - 6 - thia - 1 - azabicyclo[4.2.0]oct - 2 - èn - 2 - carboxylate - pivalate ainsi que de sels de cet ester et d'hydrates de cet ester ou de ces sels, caractérisé en ce qu'on fait réagir l'acide (6R, 7R) - 7 - [2 - (2 - Amino - 4 - thiazolyl) - 2 - (Z - méthoxy - imino) - acétamido] - 3 - /[(2,5 - dihydro - 6 - hydroxy - 2 - méthyl - 5 - oxo - as - triazin - 3 - yl)thio] - méthyl/ - 8 - oxo - 5 - thia - 1 - azabicyclo[4.2.0]oct - 2 - èn - 2 - carboxylique, de préférence sous la forme du sel disodique, avec un halogénure de pivaloyloxyméthyle, de préférence avec l'iodure, et si on le désire en ce qu'on transforme le produit obtenu en un sel ou un hydrate ou un hydrate de ce sel.